(19) Europäisches Patentamt
European Patent Office
Office européen des brevets

(11) EP 0 941 979 B1

(12) EUROPÄISCHE PATENTSCHRIFT

(45) Veröffentlichungstag und Bekanntmachung des Hinweises auf die Patenterteilung:
**05.06.2002 Patentblatt 2002/23**

(51) Int Cl.[7]: **C07C 29/56**, C07C 33/02

(21) Anmeldenummer: **99104411.6**

(22) Anmeldetag: **05.03.1999**

(54) **Kontinuierliches Verfahren zur Herstellung von Dimethylvinylcarbinol durch Isomerisierung von Prenol**

Continuous method for the preparation of dimethylvinylcarbinol by isomerisation of prenol

Procédé continu pour la préparation de diméthylvinylcarbinol par isomérisation de prénol

(84) Benannte Vertragsstaaten:
**AT BE CH DE DK ES FI FR GB GR IE IT LI NL PT SE**
Benannte Erstreckungsstaaten:
**RO SI**

(30) Priorität: **12.03.1998 DE 19810669**

(43) Veröffentlichungstag der Anmeldung:
**15.09.1999 Patentblatt 1999/37**

(73) Patentinhaber: **BASF AKTIENGESELLSCHAFT**
**67056 Ludwigshafen (DE)**

(72) Erfinder:
- **Kiefer, Matthias Dr.**
  **69226 Nussloch (DE)**
- **Siegel, Wolfgang Dr.**
  **67117 Limburgerhof (DE)**
- **Therre, Jörg Dr.**
  **67550 Worms (DE)**
- **Pahl, Melanie**
  **68307 Mannheim (DE)**
- **Aquila, Werner Dr.**
  **68309 Mannheim (DE)**
- **Schäfer-Lüderssen, Ulrich Dr.**
  **67063 Ludwigshafen (DE)**

(56) Entgegenhaltungen:
**EP-A- 0 860 415**
**DE-A- 1 925 197**
**DE-A- 2 056 343**
**GB-A- 411 741**
**US-A- 2 435 078**
**US-A- 3 355 505**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein kontinuierliches Verfahren zur Herstellung von Dimethylvinylcarbinol durch Isomerisierung von Prenol in wäßriger Lösung in Gegenwart von Protonsäuren.

Dimethylvinylcarbinol (DMVC; 2-Methyl-3-buten-2-ol) ist ein wichtiges Zwischenprodukt der industriellen organischen Chemie und dient insbesondere als Zwischenprodukt für die Herstellung von Riechstoffen oder auch als Additiv in Seifen und Detergentien.

Es ist bekannt, daß 3-Methyl-2-buten-1-ol (Prenol) unter Säurekatalyse zu DMVC isomerisieren kann. Diese Isomerisierung entspricht einer 1,3-Wanderung der Hydroxygruppe und einer entsprechenden Verschiebung der Doppelbindung, wie in der folgenden Gleichung dargestellt ist:

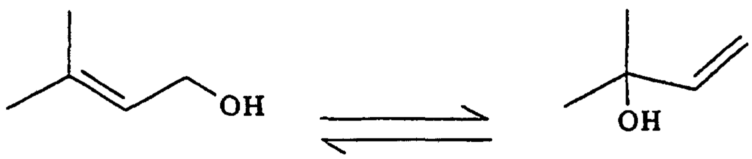

PRENOL DMVC

Diese Wanderung einer Doppelbindung und eines Substituenten ist für Allylverbindungen bekannt und wird im allgemeinen als Allyl-Umlagerung bezeichnet. Allyl-Umlagerungen von Allylalkoholen sind Gleichgewichtsreaktionen.

Eine allgemeine Übersicht über die durch Protonensäuren katalysierte Isomerisierung von Allylalkoholen findet sich beispielsweise in Houben-Weyl: "Methoden der organischen Chemie", Band VI, 1b, Seite 528ff, Stuttgart, 1984. Dort wird unter anderem beschrieben, daß sich besonders einfach solche Isomerisierungen durchführen lassen, bei denen aus einem tertiären Allylalkohol mit endständiger C=C-Doppelbindung der entsprechende primäre Alkohol mit innenständiger C=C-Doppelbindung entsteht.

Im Einklang mit diesem Wissen wurde in der Vergangenheit industriell vor allem die Isomerisierung von DMVC, einem tertiären Allylalkohol, zu seinem Isomeren, Prenol, einem primären Allylalkohol, genutzt. Beide Verbindungen wurden beispielsweise in der Riechstoffindustrie als Grundstoff zur Herstellung von Alkoholen der Terpenreihe verwendet. Auf Basis der früher weit verbreiteten technischen Carbid-Acetylenchemie waren Dialkyl-Alkenylcarbinole, wie das DMVC, durch baseninduzierte Kondensation von 1-Alkinen, wie dem Acetylen, mit Ketonen, wie Aceton, und nachfolgende Hydrierung der Dreifach- zur Doppelbindung leicht zugänglich. Heutzutage ist Acetylen ein vergleichsweise seltener und teurer Rohstoff. Auf der heute üblichen petrochemischen Rohstoffbasis sind dagegen Olefine wie Dialkylalkene, wie Isobuten, leicht zugänglich. Diese können durch Kondensation mit Aldehyden, wie dem Formaldehyd, und nachfolgende Isomerisierung der Doppelbindung leicht zu Prenol oder Prenol-Derivaten, wie mit organischen Resten substituierten Prenolen, verarbeitet werden. Technisch besteht daher heute vor allem Bedarf an einem Verfahren, mit dessen Hilfe aus dem primären Allylalkohol Prenol der tertiäre Allylalkohol DMVC hergestellt werden kann.

In der SU-A 181 090 wird ein Verfahren zur Herstellung von DMVC durch Dehydratation von 3-Methyl-1,3-butandiol beschrieben, bei dem eine Mineralsäure, insbesondere Schwefelsäure, in einer Konzentration unterhalb von 1 Gew.-% (entsprechend einem pH-Wert oberhalb von 0,7), vorzugsweise in einer Konzentration von 0,3 bis 0,5 Gew.-%, als Katalysator eingesetzt wird. Dieser bevorzugte Konzentrationsbereich entspricht einem pH-Bereich von 1,0 bis 1,2. Diese Schrift lehrt auch, daß bei diesem Verfahren auch die isomeren Alkohole $C_5H_9OH$ (3-Methyl-3-buten-1-ol und Prenol) als Ausgangsstoffe verwendet werden können. Außerdem ergibt sich aus dem vorgelegten Beispiel,daß bei der Durchführung des offenbarten Verfahrens in hohen Mengen Nebenprodukte anfallen, die regelmäßig diskontinuierlich aus dem Reaktor entfernt werden müssen. Die Ausbeute an DMVC aus 3-Methyl-1,3-butandiol beträgt nur 70%. Ausbeuteangaben für die erwähnte Verfahrensvariante ausgehend von Prenol sind nicht enthalten. Ebenso werden keine Angaben über den Zusammenhang zwischen Säurekonzentration, Reaktionsvolumen und Zuflußmenge an frischem Prenol gemacht.

A.I. Lebedeva und L.L. Schukovskaya, untersuchten gemäß J. Gen. Chem. USSR, 21 (1951) Seiten 1235 - 1241, die Abhängigkeit der Isomerisierung von DMVC vom pH-Wert des Reaktionsmediums und von der angewendeten Reaktionstemperatur bei einer Reaktionsdauer von 30 Stunden. Sie stellten fest, daß erst bei einem pH-Wert von 1,29 oder kleiner eine Isomerisierung des eingesetzten DMVC bei Raumtemperatur festzustellen war. Bei einem pH-Wert von 1,32 oder darüber wurde weder bei Raumtemperatur noch in einem siedenden Wasserbad irgendeine Isomerisierung des DMVC beobachtet. Das diskontinuierliche Verfahren weist eine komplizierte, großtechnisch nicht wirtschaftlich anwendbare Aufarbeitung auf.

I. N. Nazarov, I. N. Azerbaev und V. N. Rakcheeva lehren in Bull. Acad. Sci. USSR, Chem. Ser. 1946, 419 -

426, daß die Isomerisierung von Dialkylvinylcarbinolen, also den tertiären Allylalkoholen, in die entsprechenden primären Allylalkohole in einem Temperaturbereich von 60 bis 100 °C unter dem Einfluß von 0,1 gew.-%iger Schwefelsäure, also bei einem pH-Wert von etwa 1,7, "ziemlich glatt" verläuft, während bei Raumtemperatur die Isomerisierung unter Einfluß von 1 - 5 %iger Schwefelsäure, also pH-Werten von etwa 0,7 und darunter, durchgeführt wird. Diese Veröffentlichung lehrt weiterhin, daß bei einer Schwefelsäurekonzentration von 0,01 %, also einem pH-Wert von 2,7, der Isomerisationsprozeß selbst bei einer Temperatur von 100 °C zu langsam verläuft. Die bekannten Beispiele für die Isomerisierung von Allylalkoholen wurden daher sämtlich mit hohen Säurekonzentrationen, also niedrigen pH-Werten von höchstens 1,5 oder sogar deutlich unterhalb von 1 durchgeführt. Das beschrieben Verfahren ist zur wirtschaftlichen Herstellung von DMVC nicht geeignet.

In JP-A-54/061110 wird ein Verfahren zur Isomerisierung von Allylalkoholen unter Verwendung von großen Mengen an Borsäure als Katalysator beschrieben. Verwendet werden 0,1 bis 60 Gew.-%, insbesondere 1 bis 30 Gew.-% Borsäure. Umsätze oberhalb von 90 % werden erst mit Borsäurekonzentrationen oberhalb von 7 Gew.-% Borsäure erreicht, bei noch höheren Borsäuremengen sinkt die Selektivität der Isomerisierung rapide. Die erzielten Ausbeuten liegen unterhalb von 85 %. Über die Art der verwendeten Kolonne und das verwendete Rückflußverhältnis werden keine Angaben gemacht. Die Beispiele zeigen, daß das erhaltenen DMVC mit Prenol verunreinigt war. Die beschriebene Aufarbeitung ist sehr kompliziert. Mit dem beschriebenen Verfahren ist eine wirtschaftliche Herstellung von DMVC aus Prenol nicht möglich.

DE-A-1925197 offenbart ein Verfahren zur Herstellung von 2-Methyl-3-buten-2-ol durch Isomerisierung von 3-Methyl-3-buten-1-ol. Das 2-Methyl-3-buten-2-ol wird dadurch hergestellt, daß man auf 3-Methyl-3-buten-1-ol bei Temperaturen von 20 bis 250°C unter Entfernung des entstandenen 2-Methyl-3-buten-2-ol durch Abdestillieren wäßrige Säure entwickeln läßt.

Nachteilig an allen bekannten Verfahren zur Isomerisierung von Allylalkoholen war, daß bei ihnen größere Mengen an unerwünschten Nebenprodukten gebildet werden.

Es bestand daher nach wie vor ein großer Bedarf an einem Verfahren, mit dessen Hilfe eine kontinuierliche Herstellung von reinem DMVC aus Prenol auf einfache, billige und möglichst selektive Weise bei dennoch hohen Raumzeitausbeuten möglich wird.

Gegenstand der Erfindung ist dementsprechend ein Verfahren zur kontinuierlichen Herstellung von Dimethylvinylcarbinol (DMVC) durch kontinuierliches Umsetzen von Prenol mit der wäßrigen Lösung einer Protonensäure im Sumpf oder im unteren Teil einer Rektifizierkolonne unter Abdestillieren des gebildeten DMVC in Form eines azeotropen Gemisches mit Wasser, das dadurch gekennzeichnet ist, daß man

a) während der Umsetzung dafür sorgt, daß sich im Reaktionsgemisch möglichst kein flüssiges Zweiphasengemisch ausbildet,

b) das sich bildende DMVC mit einem Rücklaufverhältnis von mindestens 2 abdestilliert und

c) das Reaktionsvolumen und/oder die Menge an zugegebenem Prenol pro Stunde und/oder die Konzentration an Protonen im Reaktionsgemisch so bemißt, daß der als Verweilzeitkonzentrationskennzahl definierte Quotient aus dem Reaktionsvolumen und dem pro Stunde zugegebenen Volumen an Prenol multipliziert mit der Konzentration an Protonen im Reaktionsgemisch einen Wert zwischen 0,001 h · mol/l und 1 h · mol/l, vorzugsweise 0,01 bis 0,15 h · mol/l, aufweist.

Nur wenn man dafür sorgt, daß sich im Reaktionsgemisch möglichst kein flüssiges Zweiphasengemisch ausbildet, ist die Bildung von Nebenprodukten weitestgehend unterdrückt.

Zur Verhinderung der Bildung eines flüssigen Zweiphasengemisches im Reaktionsgemisch ist es neben der Verwendung von nicht zu großen Mengen an Prenol wichtig, daß man kontinuierlich aus dem Reaktionsgemisch im Reaktionsvolumen einen Teilstrom entnimmt, diesen über ein Phasentrenngefäß leitet, dort die gebildeten geringen Mengen an in Wasser nicht oder nur teilweise löslichen organischen Substanzen ganz oder zumindest weitgehend entfernt und danach die wäßrige Phase wieder in das Reaktionsvolumen zurückleitet.

Wichtig für die erstrebte hohe DMVC-Ausbeute ist auch das Vorliegen einer genügend hohen Heizleistung, um das sich bildende DMVC mit einem Rücklaufverhältnis von mindestens 2 abzudestillieren, wodurch in der Regel im Destillat der Kolonne praktisch kein Prenol mehr enthalten ist. Es ist vorteilhaft, daß man das DMVC mit einem Rücklaufverhältnis von 2 bis 25, vorzugsweise 4 bis 20, abdestilliert.

Zur Aufarbeitung des am Kopf der Kolonne gewonnenen Destillats kann dieses einfach mit einem mit Wasser nur teilweise mischbaren Extraktionsmittel in intensiven Kontakt gebracht und die sich bildende organische Phase von der wäßrigen Phase getrennt werden. Die abgetrennte wäßrige Phase wird mit Vorteil gleich wieder in das Reaktionsvolumen zurückgeleitet und besonders bevorzugt als Rücklauf auf den Kopf der Isomerisierungskolonne aufgebracht.

Die erhaltene organische Phase kann auf einfache Weise in einer Rektifikationskolonne aufgetrennt werden,

wobei man an deren Kopf eine Mischung aus wenig DMVC, Wasser und dem Extraktionsmittel und aus deren Sumpf reines DMVC gewinnt.

Mit besonderem Vorteil führt man die Isomerisierung in einer Kolonne mit 6 bis 40 theoretischen Trennstufen durch.

Als Einbauten für die Isomerisierungskolonne sowie die Rektifizierungskolonne sind u.a. Füllkörper, verschieden gestaltete Kolonnenböden oder aber strukturierte Packungen aus Blech oder Metallgewebe geeignet.

Das erfindungsgemäße Verfahren läßt sich besonders vorteilhaft durchführen, wenn man die wäßrige Lösung der Protonensäure je nach Reaktionsvolumen und Volumen des pro Stunde zugegebenen Prenols auf einen pH-Wert zwischen 1,2 und 3,5 einstellt.

Das Verfahren ist in der Figur 1 schematisch dargestellt und wird im folgenden beschrieben.

Das umzusetzende Prenol wird in die Kolonne 1, die sogenannte Isomerisierungskolonne, enthaltend 3 bis 55, vorzugsweise 5 bis 50, insbesondere 6 bis 40 theoretische Trennstufen, zugegeben. Die Zugabe erfolgt bevorzugt in den Sumpf der Kolonne, kann aber auch in den unteren Teil dieser Isomerisierungskolonne erfolgen. Gegebenenfalls kann es auch günstig sein, das Prenol in an den Sumpf der Kolonne angeschlossene Behälter oder Apparate zuzugeben. Gegebenenfalls kann der Sumpf der Kolonne durch einen Behälter vergrößert sein, um ein größeres Reaktionsvolumen zur Verfügung zu stellen.

Der Sumpf der Kolonne 1 ist mit einer hauptsächlich aus Wasser bestehenden Lösung gefüllt, in dem sich eine genau bemessene Konzentration an Protonen ($H^+$-Ionen) befindet. Wie groß die Konzentration an Protonen ist, kann durch Messung des pH-Wertes der Lösung oder durch Titration einfach bestimmt werden. Gegebenenfalls ist die Installation einer online-pH-Messung sinnvoll. Die Konzentration an Protonen soll gemäß dieser Erfindung so eingestellt werden, daß die Verweilzeitkonzentrationskennzahl einen Wert zwischen 0,001 h · mol/l und 1 h · mol/l und insbesondere zwischen 0,005 h · mol/l und 0,25 h · mol/l aufweist, wobei die Verweilzeitkonzentrationskennzahl (abgekürzt VKZ) definiert ist als der Quotient aus dem Reaktionsvolumen und dem pro Stunde zugegebenen Volumen an Prenol, multipliziert mit der Konzentration an Protonen:

Verweilzeitkonzentrationskennzahl = (Reaktionsvolumen / Zufluß an Prenol pro Stunde) mal der Konzentration an Protonen in Mol pro Liter).

Unter dem sogenannten Reaktionsvolumen verstehen wir hierbei das mit Flüssigkeit gefüllte Volumen der Isomerisierungskolonne, in dem Prenol, Wasser und Säure in Kontakt stehen und daher die Umlagerung zu DMVC vonstatten geht. Bei Verwendung einer unter Reaktionsbedingungen nicht flüchtigen Säure, die in den Sumpf der Kolonne zugegeben wird, ist das Reaktionsvolumen in der Regel das Volumen des Sumpfes der Isomerisierungskolonne mit angeschlossenen Verdampfern, Behältern und Rohrleitungen. Dieses Volumen kann durch Ausmessen der Apparate und Rohrleitungen einfach bestimmt werden.

Wird eine nicht flüchtige Säure oberhalb des Sumpfes der Kolonne zugegeben oder eine flüchtige Säure verwendet, kann die Isomerisierungskolonne auch als Reaktionskolonne betrieben werden, wobei dann zusätzlich auch die im Kolonnenkörper befindliche Flüssigkeit als Reaktionsvolumen dient und rechnerisch einbezogen werden muß.

Der volumetrische Zufluß an frischem Prenol zur Anlage kann durch übliche Meßinstrumente einfach und genau bestimmt werden.

Ist die Konzentration an Protonen zu niedrig, kann sie auf einfache Art durch Zugabe von Säuren oder Säuregemischen oder von Puffern erhöht werden.

Im erfindungsgemäßen Verfahren können praktisch alle Protonensäuren eingesetzt werden, die die notwendige Protonenkonzentration im Reaktionsgemisch erzeugen können, um die Verweilzeitkonzentrationskennzahl in dem beanspruchten Bereich zu halten, was die Voraussetzung für eine vorteilhafte Verfahrensführung ist. Beispielsweise seien die im folgenden aufgeführten Säuren oder Gemische dieser Säuren genannt: Flußsäure, Salzsäure, Perchlorsäure, Bromwasserstoffsäure, Schwefelsäure, Sulfonsäuren, Salze des Hydrogensulfations, wie Kalium- oder Natriumhydrogensulfat, Salpetersäure, Phosphorsäure und Salze des Dihydrogenphosphats, wie Kalium- und Natriumdihydrogenphosphat. Weitere Beispiele für verwendbare Säuren sind die stärkeren organischen Säuren wie Ameisensäure, Oxalsäure, Weinsäure, Bernsteinsäure, Maleinsäure, Fumarsäure, Adipinsäure und Citronensäure. Genauso können Salze der mehrbasigen Säuren, in denen noch saure Funktionen vorhanden sind, verwendet werden. Beispiele hierfür sind Alkalisalze mehrbasiger Carbonsäuren wie Mononatriumoxalat, Mononatriumtartrat, Mononatriumsuccinat, Mononatriumadipat, Mononatriumcitrat, Dinatriumcitrat oder die entsprechenden Kaliumverbindungen. Es ist genauso möglich, eine polymere und polyfunktionelle Säure zu verwenden. Solche Säuren sind unter der Bezeichnung saure

Ionentauscher bekannt und sind weit verbreitete Handelsprodukte. Bei Verwendung von festen Ionenaustauschern ist es vorteilhaft, diese als Festbett in einem an die Kolonne 1 angeschlossenen Schlaufenreaktor anzuordnen.

Mit besonderem Vorteil verwendet man als Protonensäuren Salzsäure, Schwefelsäure, Salpetersäure und Phosphorsäure, insbesondere Phosphorsäure, da diese preiswert ist und nicht korrosiv wirkt.

Ist die Konzentration an Protonen zu hoch, kann sie durch Zugabe von Basen erniedrigt werden.

Beispiele für einsetzbare Basen, die gegebenenfalls als wäßrige Lösungen verwendet werden können, sind: Natriumhydroxid, Kaliumhydroxid, Calciumhydroxid oder Amine, wie Alkylamine oder auch Mischungen dieser Basen.

Weiterhin kann im erfindungsgemäßen Verfahren zur Einstellung der Konzentration an Protonen eine Säure im Gemisch mit ihrer konjugierten Base, also ein sogenannter Puffer , eingesetzt werden. Geeignete Puffergemische, die sich im erfindungsgemäßen pH-Bereich für die Reaktion gut einstellen lassen, sind zum Beispiel: Phosphorsäure/Natriumphosphat, Milchsäure/Natriumlactat, Citronensäure/Natriumcitrat, Dinatriumcitrat/HCl, Kaliumhydrogenphthalat/HCl und Dinatriumhydrogenphosphat/Citronensäure.

Im Laufe des Betriebes der Isomerisierungskolonne 1 bilden sich in Wasser nicht oder nur beschränkt lösliche Nebenprodukte, deren Menge aber wegen der hohen Selektivität des Verfahrens gering ist. Diese Nebenprodukte müssen entfernt werden, wenn die Ausbeuten und Reinheiten des DMVC optimal sein sollen. Bevorzugt werden sie dadurch aus dem Verfahren entfernt, daß aus dem Reaktionsgemisch eine Teilmenge entnommen und in das Phasentrenngefäß 2 gegeben wird. Dort scheiden sich die Nebenprodukte als obere Phase ab, werden entnommen und z.B. durch Verbrennung entsorgt. Die von den Nebenprodukten weitgehend befreite wäßrige Phase kann wieder in die Isomerisierungskolonne 1 zurückgegeben werden.

Der Sumpf der Isomerisierungskolonne wird über den Verdampfer 3 mit Dampf beheizt und die die Isomerisierungskolonne am Kopf verlassenden Dämpfe (Brüden) werden im Kondensator 4 kondensiert. Das sich bildende DMVC wird mit Vorteil bei einem Rückflußverhältnis von 2 bis 25, vorzugsweise 4 bis 20, abdestilliert. Dabei soll das Rückflußverhältnis so gewählt werden, daß das am Kopf der Kolonne erhaltene Destillat kein Prenol enthält. Dies kann leicht durch gaschromatographische Analyse oder durch Beobachten des Temperaturprofils des Isomerisierungskolonne 1 festgestellt werden.

Die am Kopf der Isomerisierungskolonne gewonnenen Dämpfe (Brüden), bzw. das daraus durch Kondensation gewonnene Destillat besteht im allgemeinen aus etwa 74 Gew.-% DMVC, etwa 25 Gew.-% Wasser und etwa 1 Gew.-% Isopren und enthält kein Prenol. Das Destillat wird zur Aufarbeitung mit einem Strom vereinigt, der aus wenig Wasser, DMVC, Isopren und einem Extraktionsmittel besteht.

Geeignete Extraktionsmittel sind organische Lösungsmittel, die mit Wasser nicht oder nur teilweise mischbar sind und die einen Siedepunkt bei Normaldruck oberhalb von 30 °C und unterhalb von 200°C, vorzugsweise unterhalb von 120°C und insbesondere unterhalb von 100°C, aufweisen. Als Beispiele für solche Extraktionsmittel seien offenkettige, verzweigte oder cyclische Dialkylether, wie Diethylether, Dibutylether, tert.-Butylmethylether oder tert.-Butylethylether, aliphatische, alicyclische oder aromatische Kohlenwasserstoffe, wie Isopren, Pentan, Hexan, Cyclopentan, Cyclohexan, Benzol, Toluol oder die Xylole oder Gemische von Extraktionsmitteln, wie Leichtbenzine oder Kerosin, genannt. Die beiden Ströme werden im statischen Mischer 5 oder auch mit einem Rührer intensiv gemischt und das Gemisch in das Phasentrenngefäß 6 gegeben. Die sich abscheidende wäßrige Phase wird zurück in die Isomerisierungskolonne 1 gegeben. Die sich abscheidende organische Phase wird in die Rektifikationskolonne 7, die sogenannte Reinigungskolonne, mit dem Verdampfer 8 und dem Kondensator 9 gegeben und in einen Strom der aus Wasser, DMVC und dem Extraktionsmittel besteht und einen Strom , der aus reinem DMVC besteht, getrennt. Der das Extraktionsmittel enthaltende Strom wird wieder mit dem aus der Isomerisierungskolonne gewonnenen Destillat vereinigt. Die Konzentration des Extraktionsmittels in der sich im Phasenscheider 6 abscheidenden organischen Phase liegt zwischen 1 Gew.-% und 99 Gew.-%, insbesondere zwischen 20 Gew.-% und 80 Gew.-%.

Eine Variante des erfindungsgemäßen Verfahrens ist in Figur 2 schematisch dargestellt. Bei dieser Verfahrensvariante werden die Brüden der Isomerisierungskolonne und die Brüden der Reinigungskolonne in einem gemeinsamen Kondensator 4 kondensiert. Hierdurch kann der in Figur 1 dargestellte Kondensator 9 eingespart werden. Um das erfindungsgemäße Rückflußverhältnis in der Isomerisierungskolonne einzustellen, wird aus der organischen Phase des Phasentrenngefäßes 6 ein Strom entnommen und auf den Kopf der Isomerisierungskolonne gegeben.

Der Druck in der Isomerisierungskolonne liegt im allgemeinen zwischen 0,1 bar und 10 bar, vorzugsweise zwischen 0,8 und 1,5 bar.

Die Temperatur im Sumpf der Isomerisierungskolonne liegt mit Vorteil zwischen 45°C und 180°C, vorzugsweise zwischen 90°C und 110°C.

Mit Hilfe des erfindungsgemäßen Verfahrens kann DMVC aus Prenol kontinuierlich in einer sehr hohen Selektivität von bis zu 93,96 % hergestellt werden. Diese Tatsache ist um so überraschender, als der Umsatz an Prenol 100% beträgt. Die Reinheit des produzierten DMVC ist außerordentlich hoch und liegt in der Regel über 99 %, und das obwohl die Aufarbeitung des DMVC auch technisch sehr einfach zu realisieren ist. Für das Verfahren werden nur wenige übliche Apparate benötigt, die aus preiswerten und gut verfügbaren Materialien, wie Edelstahl, gebaut werden können, da die Korrosion in der Regel kein Problem darstellt. Bei dem Verfahren fallen nur geringe Mengen an Abfall

an, die einfach entsorgt werden können. Als Katalysatoren können geringe Mengen preiswerter und gut verfügbarer Säuren, wie insbesondere die Phosphorsäure, verwendet werden. Ein weiterer sehr wichtiger Vorteil besteht darin, daß das Verfahren bei gleichbleibender Selektivität an schwankende Produktionsmengen angepaßt werden kann, indem die Verweilzeitkonzentrationskennzahl durch Änderung der Protonenkonzentration, und/oder durch Änderung der pro Stunde zugefügten Prenols und/oder durch Änderung des Reaktionsvolumens im angegebenen Bereich gehalten wird. Überraschenderweise wurde festgestellt, daß die Ausbeute bei steigender Verweilzeitkonzentrationskennzahl ein Maximum durchläuft. In Figur 3 ist beispielsweise der Einfluß der Verweilzeitkonzentrationskennzahl (VKZ) auf die Ausbeute an DMVC bei einem Rücklaufverhältnis (RV) von 8+/-1 dargestellt. Durch Regelung des pH-Wertes kann dieses Maximum auch bei sich ändernden Zuflüssen an Prenol leicht eingehalten werden.

Beispiele 1 bis 27 (Kontinuierliche Laborversuche in der Variante gemäß Figur 1).

Das Reaktionsvolumen der Isomerisierungsapparatur 1 wurde zu Beginn des Experimentes mit 660 ml Wasser gefüllt und durch Zugabe von Phosphorsäure der in der Tabelle 1 angegebene pH-Wert bzw. die angegebene Wasserstoffionenkonzentration ($H^+$-Konz.) eingestellt. Der Sumpf der Kolonne wurde zum Sieden erhitzt und dann der Prenolzufluß gestartet. Die Menge an zugeflossenem Prenol und die Menge an produziertem DMVC wurden fortlaufend durch Wägen bestimmt. Als Extraktionsmittel wurde in das Phasentrenngefäß 6 am Kopf der Kolonne Cyclohexan zugegeben. Die Konzentration des Cyclohexans im Phasenscheider 6 am Kopf der Isomerisierungskolonne 1 betrug 30 Gew.-%. Die Ausbeute an DMVC wurde als Quotient aus der Masse des abgeflossenen DMVC und des zugeflossenen Prenols bestimmt. Die Reinheit des erzeugten DMVC wurde durch Gaschromatographie ermittelt. Der Umsatz an Prenol betrug 100%. Die Versuchsdauer lag zwischen 48 h und 96 h.

Die verfahrenswesentlichen Werte der Beispiele 1-27 sind in Tabelle 1 angegeben.

Beispiele 28 bis 30 (kontinuierliche Laborversuche in der Variante gemäß Figur 2)

Es wurde gearbeitet wie für die Beispiele 1-27 beschrieben wurde, nur daß als Sumpf der Isomerisierungskolonne 1 ein 41 Rührkessel verwendet wurde, der jeweils mit 3000 ml Reaktionsvolumen befüllt war, und daß entsprechend der schematischen Darstellung in Figur 2 ein gemeinsamer Kondensator für beide Kolonnen verwendet wurde.

Die verfahrenswesentlichen Angaben sind ebenfalls in Tabelle 1 dargestellt.

Tabelle 1

| Beispiel | pH-Wert | H+-Konzentration [mol/l] | Prenol-Zufluss [ml/h] | Reaktionsvolumen [ml] | Verweilzeit [h] | VKZ-Kennzahl [h·mol/l] | Rückflussverhältnis | Umwälzstrom Sumpfdekanter [l/h] | DMVC-Ausbeute | DMVC-Reinheit |
|---|---|---|---|---|---|---|---|---|---|---|
| 1 | 2.5 | 0.00316 | 117.6 | 660 | 5.61 | 0.0177 | 14.5 | 2.6 | 92.3% | 99.2% |
| 2 | 2.5 | 0.00316 | 117.6 | 660 | 5.61 | 0.0177 | 14.8 | 2.6 | 93.4% | 99.3% |
| 3 | 2.5 | 0.00316 | 117.6 | 660 | 5.61 | 0.0177 | 9.2 | 2.6 | 90.0% | 99.5% |
| 4 | 2.5 | 0.00316 | 117.6 | 660 | 5.61 | 0.0177 | 18.7 | 2.6 | 93.4% | 99.1% |
| 5 | 2.5 | 0.00316 | 117.6 | 660 | 5.61 | 0.0177 | 12.2 | 2.6 | 92.4% | 99.2% |
| 6 | 2.5 | 0.00316 | 117.6 | 660 | 5.61 | 0.0177 | 6.1 | 2.6 | 89.5% | 99.4% |
| 7 | 2.5 | 0.00316 | 117.6 | 660 | 5.61 | 0.0177 | 15.1 | 2.6 | 93.9% | 99.3% |
| 8 | 2.5 | 0.00316 | 235.3 | 660 | 2.81 | 0.0089 | 8.8 | 2.6 | 88.7% | 99.7% |
| 9 | 2.5 | 0.00316 | 235.3 | 660 | 2.81 | 0.0089 | 8.8 | 2.6 | 86.6% | 99.0% |
| 10 | 2.5 | 0.00316 | 235.3 | 660 | 2.81 | 0.0089 | 12.1 | 2.6 | 89.8% | 98.6% |
| 11 | 2.5 | 0.00316 | 235.3 | 660 | 2.81 | 0.0089 | 14.1 | 3.5 | 86.4% | 98.2% |
| 12 | 2.2 | 0.00631 | 235.3 | 660 | 2.81 | 0.0177 | 11.1 | 3.5 | 91.5% | 98.5% |
| 13 | 2.2 | 0.00631 | 235.3 | 660 | 2.81 | 0.0177 | 14.7 | 3.5 | 89.9% | 98.5% |
| 14 | 2.2 | 0.00631 | 235.3 | 660 | 2.81 | 0.0177 | 8.8 | 3.5 | 91.1% | 99.1% |
| 15 | 2.2 | 0.00631 | 235.3 | 660 | 2.81 | 0.0177 | 9.3 | 3.5 | 91.2% | 99.0% |
| 16 | 2.2 | 0.00631 | 235.3 | 660 | 2.81 | 0.0177 | 6.2 | 3.5 | 92.0% | 99.2% |

| Beispiel | pH-Wert | H+-Konzentration [mol/l] | Prenol-Zufluss [ml/h] | Reaktionsvolumen [ml] | Verweilzeit [h] | VKZ-Kennzahl [h·mol/l] | Rückflussverhältnis | Umwälzstrom Sumpfdekanter [l/h] | DMVC-Ausbeute | DMVC-Reinheit |
|---|---|---|---|---|---|---|---|---|---|---|
| 17 | 2.2 | 0.00631 | 235.3 | 660 | 2.81 | 0.0177 | 11.9 | 3.5 | 94.0% | 98.8% |
| 18 | 1.7 | 0.01995 | 176.5 | 660 | 3.74 | 0.0746 | 8.0 | 3.5 | 91.3% | 99.5% |
| 19 | 2.0 | 0.01000 | 176.5 | 660 | 3.74 | 0.0374 | 7.9 | 3.5 | 91.9% | 99.4% |
| 20 | 1.8 | 0.01585 | 176.5 | 660 | 3.74 | 0.0593 | 7.9 | 3.5 | 92.3% | 99.1% |
| 21 | 1.8 | 0.01585 | 176.5 | 660 | 3.74 | 0.0593 | 9.0 | 0.5 | 93.1% | 99.0% |
| 22 | 1.8 | 0.01585 | 176.5 | 660 | 3.74 | 0.0593 | 8.9 | 0.5 | 92.8% | 99.1% |
| 23 | 1.8 | 0.01585 | 176.5 | 660 | 3.74 | 0.0593 | 9.0 | 0.25 | 92.8% | 99.1% |
| 24 | 1.8 | 0.01585 | 176.5 | 660 | 3.74 | 0.0593 | 9.5 | 0.125 | 92.6% | 98.9% |
| 25 | 1.8 | 0.01585 | 176.5 | 660 | 3.74 | 0.0593 | 10.9 | 0.06 | 90.7% | 99.2% |
| 26 | 1.2 | 0.06310 | 176.5 | 660 | 3.74 | 0.2360 | 18.0 | 0.5 | 91.2% | 99.1% |
| 27 | 1.8 | 0.01585 | 176.5 | 660 | 3.74 | 0.0593 | 17.1 | 0.5 | 93.5% | 99.1% |
| 28 | 3.5 | 0.00032 | 117.6 | 3000 | 25.50 | 0.0081 | 16.1 | 2.6 | 92.5% | 99.7% |
| 29 | 3.5 | 0.00032 | 117.6 | 3000 | 25.50 | 0.0081 | 23.2 | 2.6 | 93.0% | 99.7% |
| 30 | 3.5 | 0.00032 | 117.6 | 3000 | 25.50 | 0.0081 | 23.1 | 2.6 | 93.4% | 99.6% |

Beispiel 31 (Technikumsversuch)

Die in diesem Beispiel verwendete Apparatur bestand aus einer Kolonne mit einem Durchmesser von 0,2 m, die mit einer 3,74 m hohen Sulzer BX-Packung gefüllt und mit einem Rückflußteiler ausgerüstet war. Aus dem Sumpf der Kolonne wurden 0,4 $m^3$/h Flüssigkeit entnommen und durch ein Phasentrenngefäß mit dem Volumen 0,06 $m^3$ geführt, in dem eine organische Phase abgeschieden wurde. Die wäßrige Phase wurde wieder in den Sumpf der Kolonne zurückgeleitet. Das Volumen des Sumpfes, des Phasentrenngefäßes und der angeschlossenen Rohrleitungen betrug insgesamt 0,104 $m^3$. Der pH-Wert im Sumpf der Kolonne wurde online überwacht und betrug 2,2. Die sich daraus errechnende Protonenkonzentration betrug 0,00631 mol/l. Der Zufluß an Prenol betrug 10 l/h. Dementsprechend betrug die Verweilzeitkonzentrationskennzahl (VKZ) 0,0656 h mol/l. Das Rückflußverhältnis war 16. Während des Experimentes wurden 340 kg Prenol in die Apparatur zugegeben und das erzeugte Destillat aufgefangen. Man erhielt 307 kg DMVC in Form einer 74,9 gew.-%igen wäßrigen Lösung. Die Ausbeute an DMVC betrug dementsprechend 90,3 % der Theorie, die Reinheit 98,5 % (wasserfrei gerechnet).

## Patentansprüche

1. Verfahren zur kontinuierlichen Herstellung von Dimethylvinylcarbinol (DMVC) durch kontinuierliches Umsetzen von Prenol mit der wäßrigen Lösung einer Protonensäure im Sumpf oder im unteren Teil einer Rektifizierkolonne (1) unter Abdestillieren des gebildeten DMVC in Form eines azeotropen Gemisches mit Wasser, **dadurch gekennzeichnet, daß** man

   a) zur Verhinderung der Bildung eines flüssigen Zweiphasengemisches im Reaktionsgemisch aus diesem kontinuierlich einen Teilstrom entnimmt, über ein Phasentrenngefäß (2) leitet, dort die im Verlauf der Umsetzung gebildeten geringen Mengen an in Wasser nicht oder nur teilweise löslichen organischen Nebenprodukten ganz oder zumindest weitgehend entfernt und die wäßrige Phase wieder in das Reaktionsvolumen zurückleitet,

   b) das sich bildende DMVC mit einem Rücklaufverhältnis von mindestens 2 abdestilliert und

   c) das Reaktionsvolumen und/oder die Menge an zugegebenem Prenol pro Stunde und/oder die Konzentration an Protonen im Reaktionsgemisch so bemißt, daß der als Verweilzeitkonzentrationskennzahl definierte Quotient aus dem Reaktionsvolumen und dem pro Stunde zugegebenen Volumen an Prenol multipliziert mit der Konzentration an Protonen im Reaktionsgemisch einen Wert zwischen 0,001 h · mol/l und 1h · mol/l aufweist.

2. Verfahren gemäß Anspruch 1, **dadurch gekennzeichnet, daß** man das Reaktionsvolumen und/oder die Menge an zugegebenem Prenol pro Stunde und/oder die Konzentration an Protonen im Reaktionsgemisch so bemißt, daß der als Verweilzeitkonzentrationskennzahl definierte Quotient aus dem Reaktionsvolumen und dem pro Stunde zugegebenen Volumen an Prenol multipliziert mit der Konzentration an Protonen im Reaktionsgemisch einen Wert zwischen 0,01 h · mol/l und 0,15 h · mol/l aufweist.

3. Verfahren gemäß Anspruch 1, **dadurch gekennzeichnet, daß** man das DMVC mit einem Rücklaufverhältnis von 2 bis 25, vorzugsweise 4 bis 20, abdestilliert.

4. Verfahren gemäß Anspruch 1, **dadurch gekennzeichnet, daß** man das am Kopf der Kolonne (1) gewonnene Destillat mit einem mit Wasser nur teilweise mischbaren Extraktionsmittel in intensiven Kontakt bringt, die sich bildende organische Phase von der wäßrigen Phase trennt und die wäßrige Phase in das Reaktionsvolumen zurückleitet.

5. Verfahren gemäß Anspruch 5, **dadurch gekennzeichnet**, das man die am Kopf der Kolonne erhaltene organische Phase in einer Rektifikationskolonne (7) auftrennt, wobei man an deren Kopf eine Mischung aus wenig DMVC, Wasser und dem Extraktionsmittel und aus dem Sumpf reines DMVC gewinnt.

6. Verfahren gemäß Anspruch 7, **dadurch gekennzeichnet, daß** man die am Kopf der Rektifikationskolonne (7) anfallende Mischung aus wenig DMVC, wenig Wasser und dem Extraktionsmittel zur Extraktion des am Kopf der Isomerisierungskolonne (1) gewonnenen Destillats verwendet.

7. Verfahren gemäß Anspruch 1, **dadurch gekennzeichnet, daß** man die Isomerisierung in einer Kolonne mit 3 bis

55 theoretischen Trennstufen durchführt.

8. Verfahren gemäß Anspruch 1, **dadurch gekennzeichnet, daß** man die wäßrige Lösung der Protonensäure auf einen pH-Wert von 1,2 bis 3,5 einstellt.

9. Verfahren gemäß Anspruch 1, **dadurch gekennzeichnet, daß** man die den Kopf der Isomerisierungskolonne (1) verlassenden Dämpfe und die den Kopf der Rektifizierungskolonne (7) verlassenden Dämpfe in einem gemeinsamen Kondensator (9) kondensiert.

**Claims**

1. A process for the continuous preparation of dimethylvinylcarbinol (DMVC) by continuously reacting prenol with the aqueous solution of a protic acid in the still or in the lower part of a rectification column (1) while distilling off the DMVC formed in the form of an azeotropic mixture with water, which comprises

   a) in order to prevent the formation of a liquid two-phase mixture in the reaction mixture, continuously taking from the latter a partial stream, passing it over a phase separation vessel (2), completely or at least largely removing there the small quantities, formed in the course of the reaction, of organic by-products which are insoluble or only partially soluble in water, and returning the aqueous phase to the reaction volume,

   b) distilling off the DMVC which forms at a reflux ratio of at least 2 and

   c) setting the reaction volume and/or the amount of prenol added per hour and/or the concentration of protons in the reaction mixture in such a manner that the quotient, defined as residence time concentration coefficient, of the reaction volume and the volume of prenol added per hour multiplied by the concentration of protons in the reaction mixture has a value between 0.001 h · mol/l and 1 h · mol/l.

2. A process as claimed in claim 1, which comprises setting the reaction volume and/or the amount of prenol added per hour and/or the concentration of protons in the reaction mixture in such a manner that the quotient, defined as residence time concentration coefficient, of the reaction volume and the volume of prenol added per hour multiplied by the concentration of protons in the reaction mixture has a value between 0.01 h · mol/l and 0.15 h · mol/l.

3. A process as claimed in claim 1, which comprises distilling off the DMVC at a reflux ratio of from 2 to 25, preferably from 4 to 20.

4. A process as claimed in claim 1, which comprises bringing the distillate obtained at the head of the column (1) into close contact with an extractant which is only partially miscible with water, separating the organic phase which forms from the aqueous phase and returning the aqueous phase to the reaction volume.

5. A process as claimed in claim 4, which comprises separating the organic phase obtained at the head of the column in a rectification column (7), a mixture of a little DMVC, water and the extractant being obtained at the head thereof and pure DMVC being obtained from the still.

6. A process as claimed in claim 5, which comprises using the mixture of a little DMVC, a little water and the extractant, which is produced at the head of the rectification column (7), for extracting the distillate obtained at the head of the isomerization column (1).

7. A process as claimed in claim 1, which comprises carrying out the isomerization in a column having from 3 to 55 theoretical plates.

8. A process as claimed in claim 1, which comprises adjusting the pH of the aqueous solution of protic acid to from 1.2 to 3.5.

9. A process as claimed in claim 1, which comprises condensing the vapors leaving the head of the isomerization column (1) and the vapors leaving the head of the rectification column (7) in a common condenser (9).

## Revendications

1. Procédé pour la préparation continue du diméthylvinylcarbinol (DMVC) par réaction continue du Prénol (3-méthyl-2-butén-1-ol) avec la solution aqueuse d'un acide protonique au pied ou dans la partie inférieure d'une colonne de rectification (1) avec distillation du DMVC formé à l'état de mélange azéotrope avec l'eau, **caractérisé par le fait que**

   a) pour éviter la formation d'un mélange liquide à deux phases dans le mélange de réaction, on prélève en continu dans ce dernier un courant partiel qu'on envoie à un récipient séparateur de phases (2) dans lequel on élimine totalement ou pratiquement totalement les petites quantités de produits d'accompagnement organiques insolubles ou partiellement solubles dans l'eau formés dans le cours de la réaction et on recycle la phase aqueuse dans le volume de réaction,
   b) on distille le DMVC qui se forme avec un rapport de reflux d'au moins 2 et
   c) on règle le volume de réaction et/ou la quantité de Prénol introduite par heure et/ou la concentration des protons dans le mélange de réaction en sorte que le quotient du volume de réaction et du volume de Prénol introduit par heure multiplié par la concentration des protons dans le mélange de réaction, définissant la caractéristique durée de passage-concentration, se situe entre 0,001 h . mol/l et 1h . mol/l.

2. Procédé selon la revendication 1, **caractérisé par le fait que** l'on règle le volume de réaction et/ou la quantité de Prénol introduite par h et/ou la concentration des protons dans le mélange de réaction en sorte que le quotient du volume de réaction et du volume de Prénol introduit par h multiplié par la concentration des protons dans le mélange de réaction, définissant la caractéristique durée de passage-concentration, se situe entre 0,01 h . mol/l et 0,15 h . mol/l.

3. Procédé selon la revendication 1, **caractérisé par le fait que** l'on distille le DMVC avec un rapport de reflux de 2 à 25, de préférence de 4 à 20.

4. Procédé selon la revendication 1, **caractérisé par le fait que** l'on met le distillat obtenu en tête de la colonne (1) en contact intensif avec un agent d'extraction miscible en partie seulement avec l'eau, on sépare la phase organique qui se forme de la phase aqueuse et on recycle la phase aqueuse au volume de réaction.

5. Procédé selon la revendication 5, **caractérisé par** le fiat que l'on sépare la phase organique obtenue en tête de colonne dans une colonne de rectification (7) en tête de laquelle on obtient un mélange d'un peu de DMVC, d'eau et de l'agent d'extraction et au pied du DMVC pur.

6. Procédé selon la revendication 7, **caractérisé par le fait que** l'on utilise le mélange d'un peu de DMVC, d'un peu d'eau et de l'agent d'extraction obtenu en tête de la colonne de rectification (7) pour l'extraction du distillat obtenu en tête de la colonne d'isomérisation (1).

7. Procédé selon la revendication 1, **caractérisé par le fait que** l'on procède à l'isomérisation dans une colonne de 3 à 55 étages de séparation théoriques.

8. Procédé selon la revendication 1, **caractérisé par le fait que** l'on règle la solution aqueuse de l'acide protonique à un pH de 1,2 à 3,5.

9. Procédé selon la revendication 1, **caractérisé par le fait que** l'on condense les vapeurs quittant la tête de la colonne d'isomérisation (1) et les vapeurs quittant la tête de la colonne de rectification (7) dans un condenseur commun (9).

FIG.1

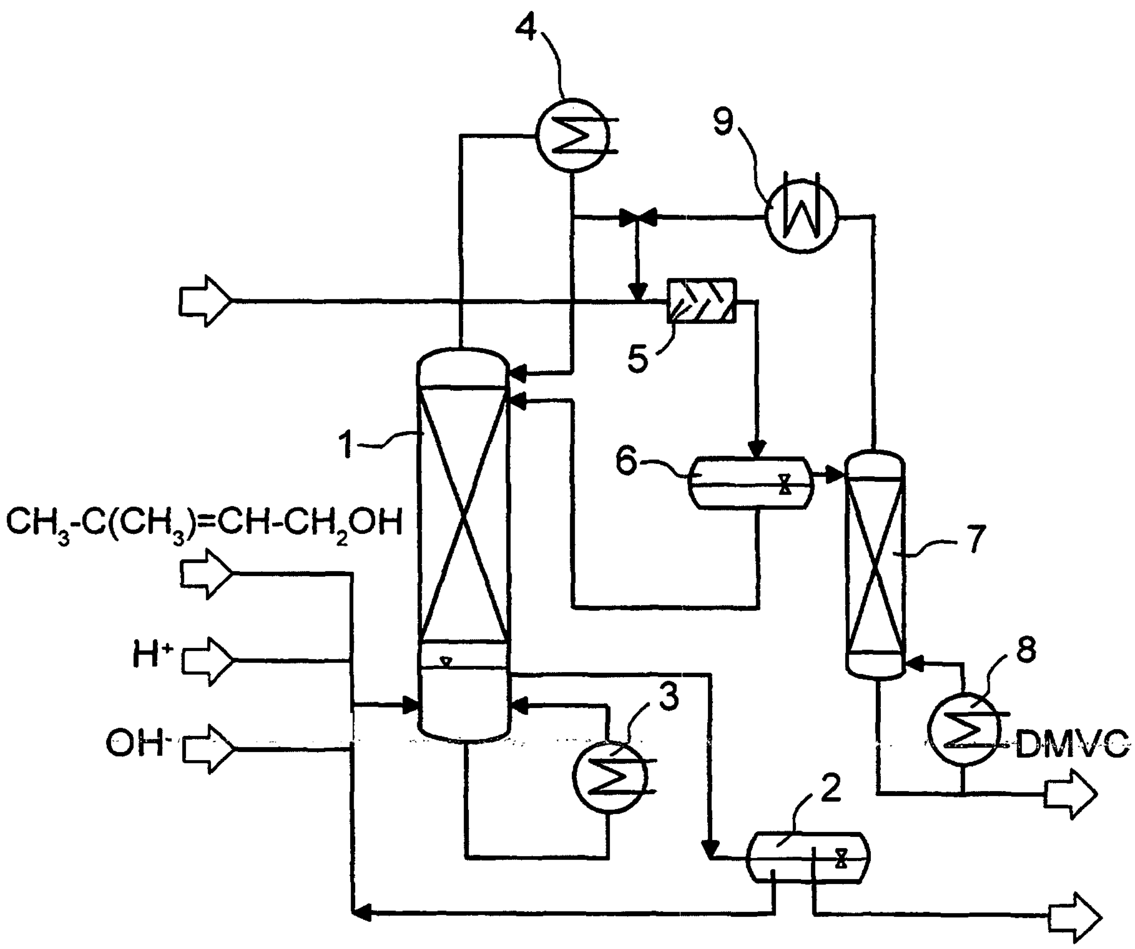

FIG.2

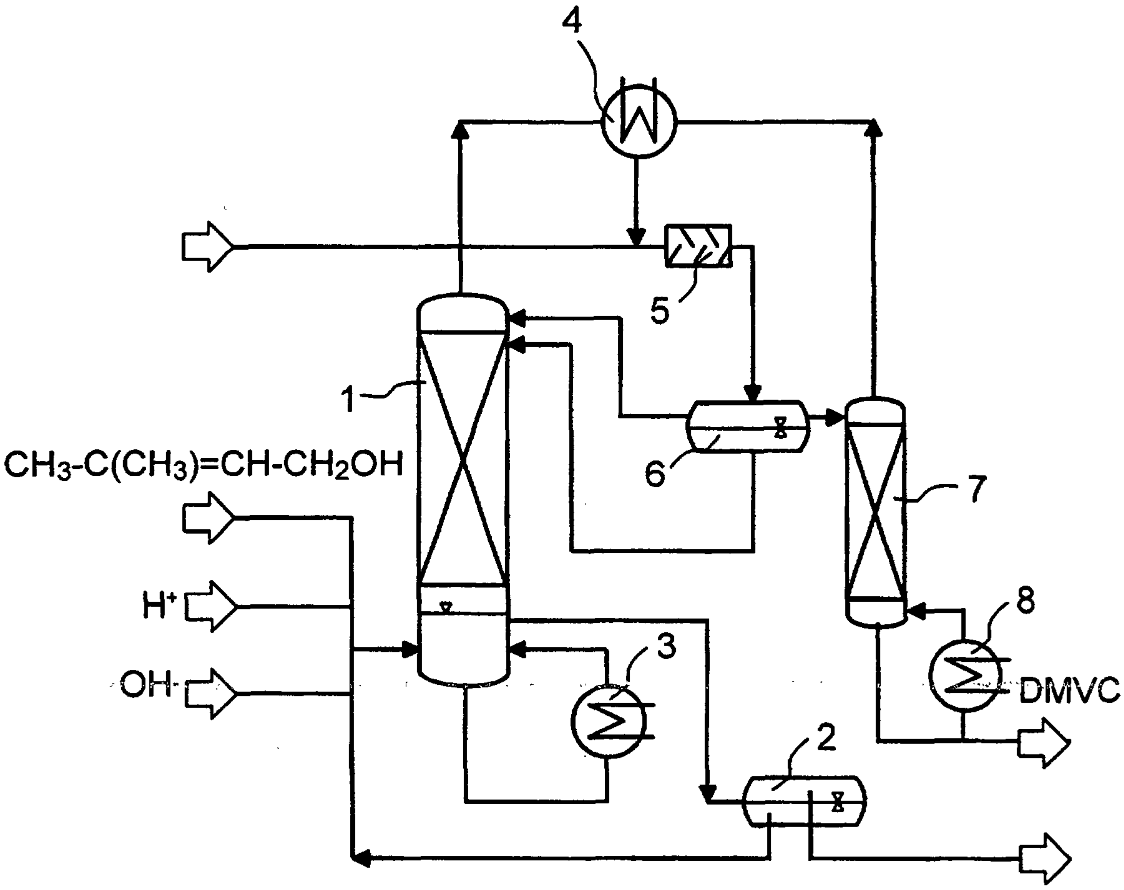

FIG.3

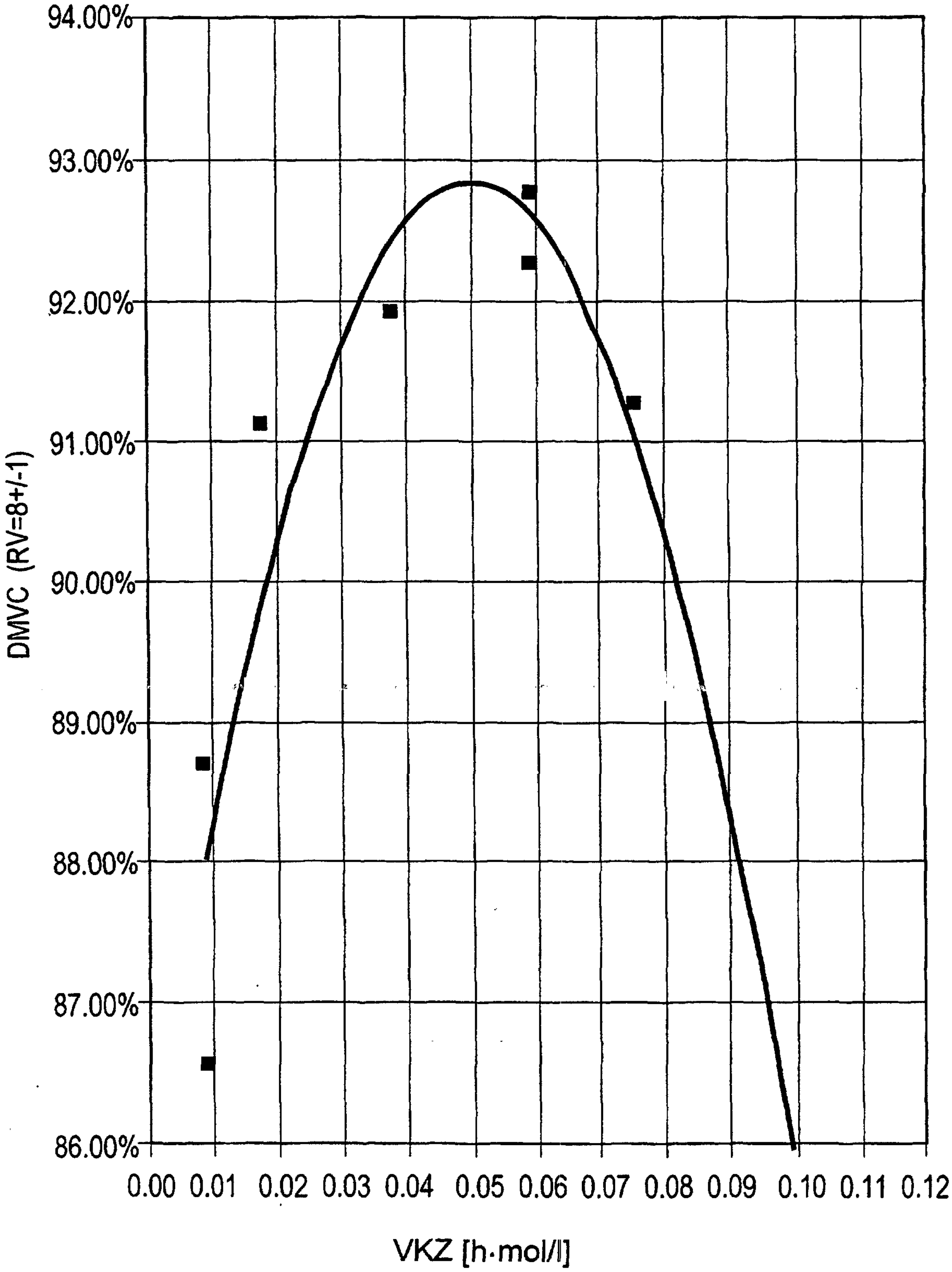
94.00%
93.00%
92.00%
91.00%
90.00%
89.00%
88.00%
87.00%
86.00%
DMVC (RV=8+/-1)
0.00 0.01 0.02 0.03 0.04 0.05 0.06 0.07 0.08 0.09 0.10 0.11 0.12
VKZ [h·mol/l]